# FASCICULE DE BREVET EUROPEEN

(11) **EP 2 445 619 B1**
(45) Date de publication et mention de la délivrance du brevet: **28.08.2013**
(21) Numéro de dépôt: 10734466.5
(22) Date de dépôt: 22.06.2010
(51) Int. Cl.: B01F 3/08, B01F 11/02

(54) **PROCÉDÉ DE PRÉPARATION D'UNE ÉMULSION HUILE-DANS-EAU STABLE**
VERFAHREN ZUR HERSTELLUNG EINER STABILEN ÖL-IN-WASSER-EMULSION
METHOD FOR PREPARING A STABLE OIL-IN-WATER EMULSION

(30) Priorité: 24.06.2009 FR 0954323
(43) Date de publication de la demande: 02.05.2012
(73) Titulaire: SAS Genialis, 18250 Henrichemont (FR)
(72) Inventeur: DESJARDINS-LAVISSE, Isabelle, F-18250 Henrichemont (FR); DESOBRY, Stéphane, F-54740 Saint-Remimont (FR)
(74) Mandataire: Office Freylinger
(86) Numéro de dépôt international: PCT/EP2010/058848
(87) Numéro de publication internationale: WO 2010/149668

(56) Documents cités:
- DE-A1-102006 032 083
- FR-A- 1 322 103
- US-A- 2 893 707
- US-B1- 6 465 015

## Description

La présente invention concerne généralement le domaine des émulsions, et plus particulièrement un procédé de préparation d'une émulsion stable d'une phase lipidique dans une phase aqueuse qui ne nécessite pas l'ajout d'émulsifiant.

Comme on le sait, une émulsion est un mélange de deux substances liquides non miscibles (qui ne se mélangent normalement pas), comme l'eau et l'huile. Le liquide formant les gouttes est appelé la phase interne, discontinue ou dispersée, tandis que le liquide entourant les gouttelettes est appelé phase continue ou externe. Dans une émulsion huile dans eau, l'huile est dispersée dans l'eau sous forme de petites gouttelettes.

Qu'il s'agisse d'une émulsion huile-dans-eau (H/E) ou eau-dans-huile (E/H), une émulsion est typiquement instable et tend à se séparer ou coalescer. Ainsi, pour que le mélange des deux phases non miscibles reste stable, on ajoute un émulsifiant.

Le principe de base pour obtenir une émulsion H/E consiste donc généralement à disperser finement la phase lipidique dans l'eau en présence d'un émulsifiant.

Cette dispersion est en fait obtenue en soumettant le mélange des phases aqueuse et lipidique, avec l'émulsifiant, à une agitation forte avec broyage, c'est-à-dire sous fort cisaillement, ce qui peut typiquement se faire par :
- un broyeur colloïdal ;
- un émulsionneur/homogénéisateur haute pression ;
- un émulsionneur à ultrasons.

Outre l'ajout d'émulsifiant, un autre facteur important pour la stabilité d'une émulsion est la taille des particules de la phase discontinue : plus la taille de ces particules est petite, plus l'émulsion est stable dans le temps.

Ainsi, au niveau industriel l'émulsionneur/homogénéisateur haute pression est largement employé car il permet d'obtenir des tailles de particules très petites, à savoir de l'ordre de 200 nm (voire moins). Dans un tel appareil, une pompe à pistons plongeurs pousse le mélange à émulsionner à travers un orifice réglable, appelé groupe homogénéisant. Plus l'orifice est faible, plus la pression affichée est élevée. Au passage de cet obstacle le fluide subit différentes contraintes produisant un cisaillement important qui génère la formation de micelles ou globules fins dispersés dans la phase continue. De nombreux secteurs industriels utilisent aujourd'hui ces appareils, capables d'atteindre 1 500 à 3 000 bars en production continue, telles l'industrie des cosmétiques, l'industrie pharmaceutique et les industries alimentaires (ingrédients, boissons, etc...).

Dans le domaine agroalimentaire, l'utilisation d'ondes dans les fréquences ultrasonores a été proposée par exemple pour la préparation de nano-émulsions dans un article intitulé « The use of ultrasonics for nanoemulsion preparation » par S. Kentisch et al. (Innovative Food Science and Emerging Technologies 9 (2008) 170-175). Le procédé décrit utilise un transducteur à ultrasons à une fréquence de 20 à 24 kHz. L'émulsion H/E obtenue présente une taille moyenne de gouttelettes de l'ordre de 135 nm en utilisant un mélange d'huile de lin et d'eau, en présence de surfactant (Tween 40).

D'autres essais de préparation d'émulsions avec des produits de synthèse ont été réalisés dans le domaine des peintures ou des polymères. Dans l'ensemble, les procédés de préparation d'émulsions par application d'ultrasons (ou « sonication ») exploitent le phénomène de cavitation qui permet une émulsification rapide sous fort cisaillement.

On l'a dit, la stabilité est un aspect important des émulsions, surtout lorsqu'elles sont transportées et stockées avant leur utilisation, deux paramètres influents à cet égard étant la taille de la phase discontinue et l'émulsifiant.

Or, pour certaines applications, on souhaiterait pouvoir se passer d'émulsifiant. Dans le domaine dermatologique et cosmétique par exemple, les émulsions du type huile-dans-eau sont aujourd'hui très appréciées pour leur confort d'utilisation et leur fraîcheur. Afin de s'affranchir de l'emploi de tensioactifs pour stabiliser ce type d'émulsions, lesquels peuvent se montrer irritants pour certains types de peaux, le EP 0 692 237 propose l'utilisation de particules thermoplastiques creuses expansées de polymère ou copolymère d'acrylonitrile assurant la dispersion de la phase huileuse.

Un autre domaine pour lequel la possibilité de préparer des émulsions sans émulsifiants ou stabilisants conventionnels trouve un grand intérêt est celui de l'agroalimentaire. La suppression d'émulsifiants permet de simplifier les recettes et d'éliminer les problèmes d'allergie ou d'intolérance liés à ce type de composés, ce qui est particulièrement bénéfique dans le développement de produits de la gamme « nutrition/santé ».

Le DE 10 2006 032 083 A1 décrit un procédé de fabrication de microcapsules contenant un agent lipophile. Le procédé implique la mise en oeuvre d'émulsions multiples, dont une étape d'émulsification d'une émulsion H/E dans une phase lipidique au moyen d'ultrasons. Les exemples décrits visent des fréquences ultrasonores de l'ordre de 20 kHz à 40 kHz.

Le FR 1 322 103 concerne un procédé de production d'émulsions H/E qui consiste à mélanger mécaniquement la substance huileuse et le liquide aqueux, à émulsionner ledit mélange à une température appropriée (par ex. 60°C) par des vibrations supérieures ou égales à 10 kHz, de préférence comprises entre 15 et 30 kHz, puis à injecter goutte à goutte dans le sens convenable, ladite émulsion dans la zone de focalisation d'un bol piézo-électrique fonctionnant entre 800 kHz et 1 MHz.

Ces deux derniers procédés sont donc complexes, emploient des traitement ultrasonores avec cavitation, et requièrent des manipulation multiples des phases à émulsionner, en des volumes variés.

### Objet de l'invention

Le but de la présente invention est donc de proposer un procédé de préparation d'émulsions stables durant plusieurs semaines à plusieurs mois qui ne requiert pas d'émulsifiant et qui soit simple à mettre en oeuvre.

### Description Générale de l'invention

Conformément à la présente invention, on propose un procédé de préparation d'une émulsion H/E stable, dans lequel on combine une phase lipidique (ou lipophile) avec une phase aqueuse (ou hydrophile), caractérisé en ce qu'on soumet le mélange de ces phases à une énergie vibratoire, dans un récipient fermé, par application d'un transducteur opérant à une fréquence supérieure ou égale à 900 kHz, de préférence supérieure à 1 MHz.

Ce traitement vibratoire haute-fréquence peut être typiquement poursuivi jusqu'à l'obtention de la concentration finale désirée en phase lipidique et/ou de la granulométrie finale désirée, ou plus simplement jusqu'à dispersion complète de la phase lipidique ; après quoi l'émulsion peut être recueillie.

Durant sa mise au point, le procédé a montré, de manière surprenante, qu'il est possible de réaliser une émulsion huile-dans-eau stable sans ajout d'émulsifiant, en soumettant le mélange à des ondes causées par application d'un transducteur opérant à une fréquence supérieure ou égale à 900 kHz. De préférence, le transducteur opère dans une plage de fréquences définie par une borne inférieure de l'ordre de 900 KHz à 1 MHz et par une borne supérieure de l'ordre de 3 MHz. Une plage plus resserrée peut s'étendre jusqu'à 1,5 MHz.

Le procédé selon l'invention permet d'obtenir des émulsions stables avec une granulométrie fine et contrôlée, les particules de la phase discontinue ayant une taille micrométrique ou nanométrique. La stabilité de ces émulsions peut aller jusqu'à plusieurs mois, et convient donc pour un usage industriel. Elles sont, en outre, immédiatement et infiniment solubles dans tout autre milieu aqueux.

De préférence, le traitement vibratoire à l'aide du transducteur est mis en oeuvre jusqu'à l'obtention d'une granulométrie micrométrique de la phase discontinue. En particulier, on vise un diamètre moyen des particules (globules) de la phase discontinue qui soit inférieur 50 µm, de manière plus préférée inférieur à 20 µm. Conventionnellement, dans le cadre d'une distribution gaussienne, le diamètre moyen correspond à la moyenne des diamètres des globules gras mesurés en volume dans l'émulsion.

Sans intention de se limiter par une explication théorique, on considère que l'application d'une énergie vibratoire haute fréquence (900 kHz et plus) au moyen d'un transducteur pour la réalisation d'émulsions présente l'avantage de faire disparaître le phénomène de cavitation généralement utilisé pour son intensité de cisaillement. En effet, les gammes de fréquence classiquement employées comprises entre 20 et 200 kHz, et généralement inférieures à 80 kHz, conduisent à la formation de bulles de cavitation dans lesquelles la température locale s'accroît jusqu'à plusieurs centaines de degrés Celsius et où la pression augmente très fortement. Cette cavitation cisaille fortement le mélange ce qui permet l'émulsification rapide mais provoque l'altération physico-chimique et biochimique du mélange. L'utilisation de fréquences hautes conformément à la présente invention annule ces altérations et préserve beaucoup mieux le produit, permettant l'obtention d'émulsions stables. On considère notamment que le présent procédé s'appuie sur un phénomène de brumisation en vase clos qui conduit à une re-solubilisation des micro/nanogouttelettes formées à la surface (causées par les ondes haute-fréquence du transducteur, de préférence du type piézoélectrique) et une émulsification progressive du mélange. Ces globules gras peuvent avoir des dimensions micrométriques (voire nanométriques) en fonction du temps d'application du procédé.

Un autre phénomène mis en oeuvre dans l'invention est une légère acidification de l'émulsion obtenue, qui semble indiquer une ionisation partielle de l'émulsion. Une telle ionisation intervenant par le jeu de liaisons électrostatiques, cela permet de conforter davantage la stabilité de l'émulsion et de se passer d'émulsifiant dans la formulation.

Ainsi, comparativement à l'émulsification ultrasonore (basse fréquence) par cavitation qui se fait sous très fort cisaillement avec les inconvénients y afférents, le présent procédé est un procédé d'émulsification directe qui permet d'obtenir une émulsion stable et fine. Si la mise en émulsion est plus longue (typiquement une à plusieurs heures) que dans les procédés par cavitation, le présent procédé a l'avantage de garantir l'intégrité des produits.

Le présent procédé est d'application générale dans tous les secteurs de l'industrie, et trouve une application particulièrement intéressante lorsque les émulsifiants peuvent poser des problèmes de confort, d'irritation, d'allergie ou d'intolérance comme par exemple dans les secteurs de l'agroalimentaire, la dermatologie, la cosmétique, les produits de relaxation et la pharmacie. D'autres secteurs intéressants sont par exemple la peinture ou les polymères.

Bien entendu, la suppression d'émulsifiants permet de réduire les coûts, et présente donc un attrait dans toutes les préparations d'émulsions à l'échelle industrielle.

On le comprendra, la composition des phases dépendra typiquement du secteur concerné. La formulation du mélange à émulsionner pourra en outre comprendre un ou plusieurs additifs. L'ajout d'émulsifiant étant possible mais n'étant pas requis, on l'évitera de préférence. Les deux phases formulées peuvent être simplement combinées dans le récipient de traitement, mais peuvent également faire l'objet d'un pré-mélange.

De manière générale, le terme phase lipidique ou lipophile désigne toutes les substances huileuses, liquides à la température de mise en oeuvre du procédé, naturelles, végétales ou animales, ou synthétiques ayant ou non une ou plusieurs activités biologiques avérées, et insolubles dans l'eau (moins de 2% en poids à température ambiante). A titre illustratif et non limitatif de ces lipides, on peut notamment citer :
- pour l'agroalimentaire : les huiles végétales (olive, tournesol, colza, arachide, mélanges d'huiles végétales,...) ; les huiles animales (poisson...), les beurres...
- pour la dermatologie / cosmétique : les huiles d'avocat, d'argan et autres huiles végétales, les huiles essentielles.

Le terme phase aqueuse (ou hydrophile) désigne toute phase contenant de l'eau et/ou de l'alcool. On peut citer l'eau adoucie ou non, minérale ou non, l'eau de source.

Par ailleurs, le mélange à émulsionner, peut comprendre des additifs. Ces additifs peuvent être ajoutés à l'une des phases, selon qu'ils sont liposolubles ou hydrosolubles. Le cas échéant, on pourra au préalable solubiliser un tel additif dans un solvant. A titre d'exemple non exhaustif, dans le secteur alimentaire, des biomolécules d'intérêt pourraient être ajoutées dans la phase aqueuse (peptides, vitamines, flavonoïdes,...) ou dans la phase lipidique (triacylglycérols, acides gras, arômes,...)

Dans la pratique, le mélange à émulsionner est placé dans un récipient maintenu fermé pendant l'application des ondes hautes-fréquences. Ces ondes sont de préférence appliquées au mélange dans la partie basse du récipient.

Les transducteurs du type piézoélectriques sont particulièrement préférés ; ils sont adaptés pour un fonctionnement stable dans la gamme de fréquence choisie et leur technologie de fabrication est bien maîtrisée. En outre, les éléments de céramique piézoélectriques peuvent être directement en contact avec, ou immergés dans, le milieu à traiter. Alternativement le transducteur peut comprendre un élément vibrant mécaniquement couplé aux céramiques piézoélectriques, lequel est alors mis en contact avec, ou immergé dans, le milieu à traiter.

Les vibrations sont appliquées de préférence pendant le temps nécessaire pour disperser entièrement la phase lipidique et atteindre la distribution granulométrique désirée. Les durées requises peuvent être facilement estimées par l'homme du métier sur bases d'essais préalables, prenant en compte les phases en présence, les volumes, et la puissance appliquée.

On notera qu'il est possible d'utiliser plusieurs transducteurs simultanément pour réaliser une même émulsion, ce qui permet notamment de réduire le temps nécessaire à l'émulsification.

Pour une bonne maîtrise du procédé, la proportion de phase lipidique est préférablement de l'ordre de 0,01 à 40 % du poids total du mélange (émulsion), de manière plus préférée la part de phase lipidique n'excède pas 30 % (m/m) du poids total, ou mieux n'excède pas 20 % (m/m).

Dans certains cas, selon la durée de traitement, on peut observer une augmentation de température du mélange induite par le fonctionnement en continu des transducteurs. Pour palier une telle augmentation de température, on maintiendra de préférence le mélange dans une plage de température prédéterminée, de préférence dans une plage de température entre 20 et 30°C. Cela peut se faire aisément en munissant le récipient fermé d'une double paroi refroidie, ou par tout autre moyen approprié. Un tel refroidissement est particulièrement intéressant si le mélange à émulsionner contient des produits thermosensibles ou volatils, tels que certains arômes d'huiles essentielles par exemple.

Selon un autre aspect de l'invention, on propose une émulsion E/H stable, exempte d'émulsifiant, présentant une granulométrie micrométrique ou nanométrique. Cette émulsion présente une stabilité de plusieurs semaines, typiquement d'au moins 3 mois à température ambiante. Une telle émulsion est avantageusement obtenue par le présent procédé. Encore un autre aspect de l'invention concerne une composition ou produit pour usage pharmaceutique, dermatologique ou alimentaire comprenant une émulsion obtenue selon le présent procédé.

Selon encore un aspect de l'invention, on propose l'utilisation du présent procédé pour la préparation de compositions dermatologiques ou pharmaceutiques ou de produits alimentaires.

Selon un ultime aspect, la présente l'invention concerne dispositif d'émulsification comprenant un récipient fermé apte à recevoir un mélange de phase lipidique et de phase aqueuse à émulsionner. Un transducteur est agencé de sorte à appliquer des vibrations au mélange dans la partie inférieure du récipient fermé, le transducteur étant configuré et piloté par une électronique de commande appropriée pour opérer dans une plage de fréquences comprises entre 900 kHz et 3 MHz.

De préférence, le dispositif comprend des moyens pour maintenir le mélange dans ledit récipient fermé à une température comprise dans une plage de température prédéterminée, de préférence dans une plage de température entre 20 et 30°C.

### Description détaillée à l'aide des figures

D'autres particularités et caractéristiques de l'invention ressortiront de la description détaillée de quelques modes de réalisation avantageux présentés ci-dessous, à titre d'illustration, en se référant aux dessins annexés. Ceux-ci montrent:
- Fig.1:: un schéma illustrant le principe d'émulsification du présent procédé en trois étapes : a) état initial, b) transducteur en marche, et c) état final ;
- Fig.2:: un graphique montrant la distribution de tailles de la phase lipidique selon l'exemple 1 ; .
- Fig.3:: un graphique montrant la distribution de tailles de la phase lipidique selon l'exemple comparatif 1 ;

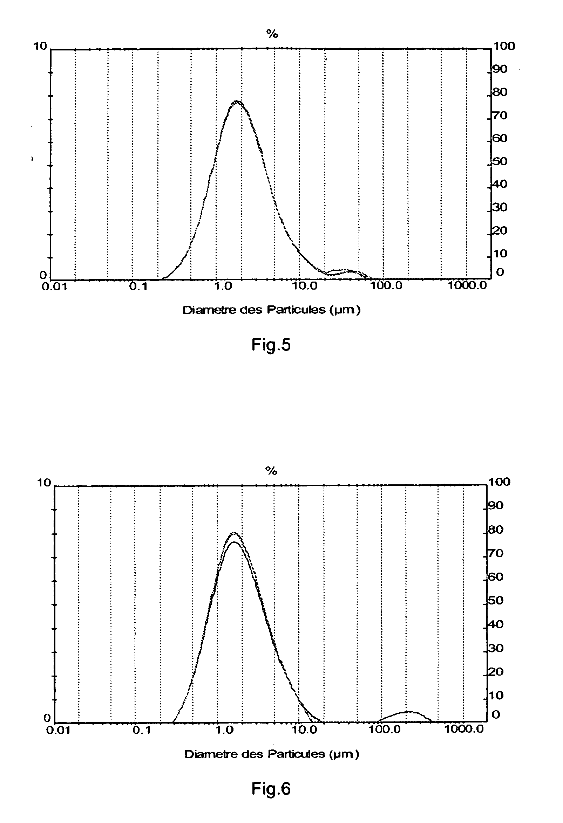

- Figs.4 à 7 :: des graphiques montrant la distribution de tailles de la phase lipidique pour les autres émulsions référencées 2 à 5 ;
- Fig.8 :: une vue en perspective d'une variante de cuve pour la mise en oeuvre du présent procédé.

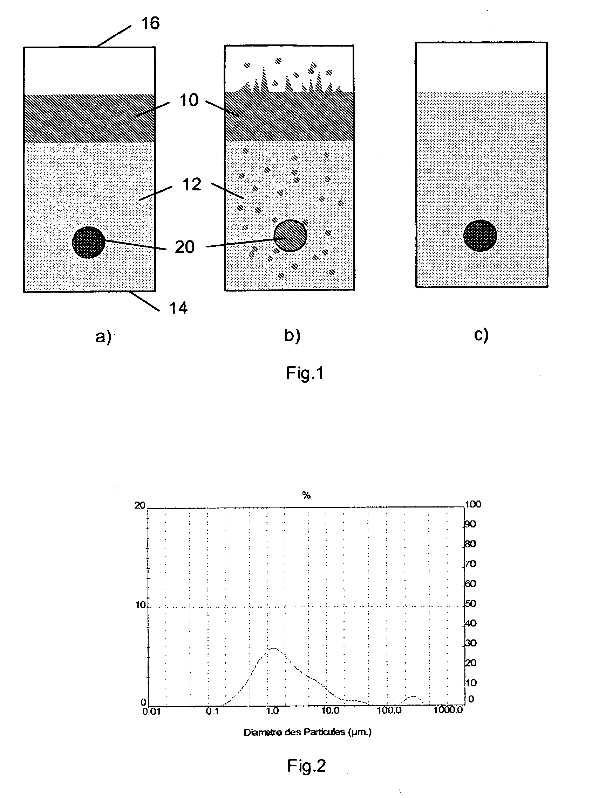

Le principe du présent procédé est représenté schématiquement à la Fig.1. Un mélange biphasique comprenant une phase lipidique 10 et une phase aqueuse 12 est préparé et placé dans un récipient 14. Dans la configuration de la Fig.1 a), la phase aqueuse 12 a été versée en premier, puis la phase lipidique 10. Le récipient 14 est ensuite fermé par un couvercle 16. Le signe de référence 20 indique un transducteur piézo-électrique opérant à une fréquence supérieure à 900 kHz, mise en oeuvre après fermeture du récipient 14. Ce transducteur piézo-électrique 20 permet d'appliquer une énergie vibratoire au mélange essentiellement sans cavitation, ce qui conduit à la formation de fines gouttelettes en surface (brumisation), et qui, en retombant dans la solution, conduit à la formation progressive d'une émulsion très fine, stable et homogène. En effet, une émulsion de taille micrométrique (voire nanométrique) est obtenue en opérant le transducteur pendant une durée variant de quelques minutes à plusieurs heures, suivant les phases et volumes considérés. Le traitement vibratoire est interrompu après l'obtention de la concentration finale désirée en phase lipidique et/ou de la granulométrie finale désirée, ou plus simplement lorsque la dispersion complète de la phase lipidique et atteinte ; après quoi l'émulsion peut être recueillie. De préférence le traitement vibratoire est arrêté après obtention de la distribution granulométrique finale désirée.

On notera que le transducteur piézoélectrique 20 est immergé dans le mélange à traiter, en partie basse du récipient 14, et préférence au centre (vu en coupe horizontale) du récipient. Il peut être situé dans la moitié inférieure du volume à traiter, et préférablement dans le premier tiers. Dans la présente variante, les céramiques piézoélectriques sont directement en contact avec le mélange à traiter. Si dans la Fig.1 on matérialise un unique transducteur 20, on pourra également utiliser plusieurs transducteurs opérants aux fréquences prescrites distribués dans le récipient, ce qui réduit la durée de traitement.

L'application du présent procédé à un simple mélange biphasique « huile + eau », sans émulsifiant, a démontré que le procédé n'altère en aucune manière l'huile utilisée (le niveau d'insaturation des chaînes d'acides gras est préservé) et que l'émulsion est stable plusieurs mois à température ambiante. L'émulsion obtenue résiste, en outre, parfaitement aux procédés de stérilisation et pasteurisation.

On notera encore que dans le présent procédé, comme illustré à la Fig.1, on met typiquement en présence dans le récipient 14 les deux phases à traiter, dans les proportions désirées pour l'émulsion à obtenir. Le traitement vibratoire est donc appliqué à un volume combiné de phases aqueuse et lipidique, dont les proportions respectives sont celles de l'émulsion désirée. Ainsi, on place dès le départ dans le récipient de traitement un volume de phase lipidique suffisant pour atteindre la concentration en phase dispersée désirée pour l'émulsion à réaliser.

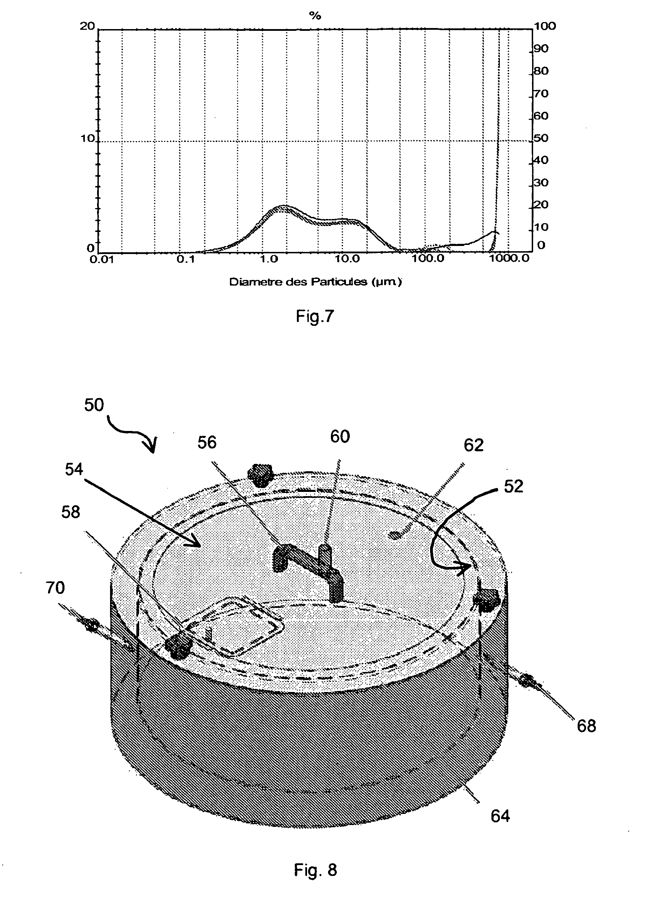

Si dans le mode de réalisation décrit ci-dessus on a versé successivement la phase aqueuse 12 puis la phase lipidique 10, on pourrait réaliser un pré-mélange dans les mêmes proportions, par exemple par agitation, avant de verser ce pré-mélange dans le récipient 14. La Fig.7 montre un exemple de réalisation de cuve 50 pour la mise en oeuvre du présent procédé. Elle comprend une cuve intérieure 52 définissant un volume destiné à recevoir le mélange à émulsionner, fermée par un couvercle 54. Le couvercle 54 est muni d'une poignée 56 et comprend une trappe d'inspection 58, un passage de fils 60 et une ouverture de mesure 62. Un ou plusieurs transducteurs opérant aux fréquences prescrites (non représentés) sont installés en partie inférieure de la cuve 52.

Le signe de référence 64 indique une paroi extérieure, concentrique à la paroi intérieure de la cuve 52, qui définit donc un espace annulaire 66 pour un fluide de refroidissement. Les embouts 68 et 70 seront donc utilisés respectivement comme entrée et sortie pour un fluide de refroidissement tel que l'eau ou un autre fluide adapté. Cette configuration de la cuve de traitement avec enveloppe ou double paroi permet de maintenir aisément le mélange dans la cuve dans une plage de température prédéterminée, de préférence de 20 à 30°C. Le débit et la température du fluide de refroidissement seront donc réglés en conséquence.

### Exemple 1.

Une émulsion a été réalisée par application du présent procédé dans les conditions opératoires illustrées à la Fig.1 à partir d'un mélange de 500 mL comprenant de l'eau d'Evian et 5 % (pourcentage massique) d'huile de Linchi inca par rapport au poids total du mélange (sans émulsifiant). Le procédé est mis en oeuvre dans une pièce à 20°C, et le transducteur piézo-électrique a été mis sous tension pendant 10 heures.

La distribution des tailles des globules gras dans l'émulsion obtenue est montrée dans le graphique de la Fig.2. L'émulsion est fine et atteint l'échelle micrométrique, avec un diamètre moyen de 1,75 µm.

L'émulsion est stable à température ambiante pendant plusieurs semaines.

Un essai de centrifugation à 4000 tours/min pendant 20 minutes avec une accélération de 3500 g n'a provoqué aucun changement de phase. L'émulsion peut donc être considérée comme stable.

### Exemple comparatif 1.

Une émulsion a été préparée avec le même mélange eau/Linchi inca qu'à l'exemple 1 ci-dessus à l'aide d'un émulsionneur-homogénéiseur haute-pression « Emulsiflex » 1700 bar - 5 cycles à 20°C.

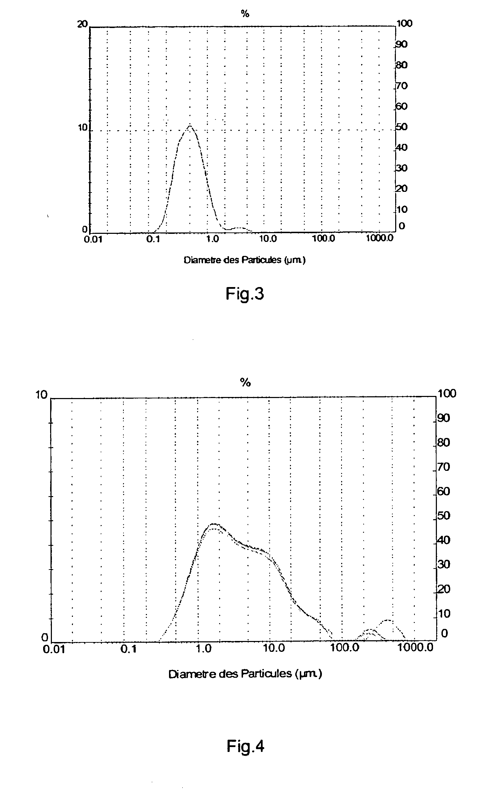

La distribution de taille des globules gras dans l'émulsion est illustrée à la Fig.3. L'émulsion réalisée est fine et atteint l'échelle nanométrique avec un diamètre moyen de 0,510 µm. L'émulsion est stable à température ambiante pendant plusieurs jours. Mais une centrifugation à 4000 tours/min pendant 20 minutes avec une accélération de 3500 g conduit à une nette séparation de phase. L'émulsion n'est donc pas stable.

On notera encore que le pH de l'émulsion obtenue ici est de 7,5 alors que le pH de l'émulsion de l'exemple 1 est de 5,85. Cette acidification semble résulter d'une légère ionisation du milieu par le procédé piézo-électrique, qui apporte une stabilisation à l'émulsion par les liaisons électrostatiques et vient donc en complément de la stabilisation obtenue par le contrôle de la taille des globules gras (phase discontinue).

### Autres exemples.

D'autres émulsions ont été préparées dans les conditions de l'exemple 1 à partir d'huiles de colza, olive et tournesol, sans aucun émulsifiant ou tensioactif (référencées 2 à 4). Une émulsion a également été préparée avec un ajout d'émulsifiant (émulsion 5). Les propriétés de ces émulsions sont résumées dans le tableau suivant, avec la référence à la Figure illustrant la distribution de taille de la phase discontinue pour chaque émulsion.

L'émulsion 2 (huile de colza) présente une distribution monomodale des globules gras avec un diamètre moyen plus élevé que précédemment (12,5 µm), mais le système est néanmoins stable pendant plusieurs semaines.

| Réf. | Mélange initial (% m/m) | Diamètre moyen des globules gras de l'émulsion | Fig. |
|---|---|---|---|
| 2 | Eau d'Evian + 5% d'huile de Colza | 12,5 µm | 4 |
| 3 | Eau d'Evian + 5% d'huile d'olive | 3,7 µm | 5 |
| 4 | Eau d'Evian + 5% d'huile de tournesol | 2,5 µm | 6 |
| 5 | Eau d'Evian + 8% d'huile de tournesol + 0.05% de twin 80 | Distribution complexe | 7 |

Les émulsions 3 et 4 (huile d'olive et tournesol) présentent une distribution des globules gras monomodale de faible taille, avec un diamètre moyen de 3,7 et 2,5 µm respectivement.

Ces émulsions ne subissent aucune séparation de phase lorsque soumises à une centrifugation dans les conditions de l'exemple 1.

Ces essais valident donc l'application du présent procédé à la préparation d'émulsions fines et stables à partir d'huiles alimentaires, sans ajout d'émulsifiant, tensioactif ou autre stabilisant conventionnel.

On remarquera encore que l'ajout de Twin 80 dans l'exemple 5, un émulsifiant très utilisé et connu pour sa forte capacité de stabilisation des émulsions, perturbe la distribution granulométrique de la phase discontinue. Il est ajouté ici avant dans le mélange, c.-à-d. avant la mise en oeuvre des ondes hautes-fréquences, comme dans le cas de la fabrication d'une émulsion courante par agitation.

Comme on le voit à la Fig.7, trois populations de globules gras différentes sont apparues. La première a un diamètre moyen de l'ordre de 2 µm, équivalent à ce qui avait été analysé en l'absence d'émulsifiant mais une 2^{ène} population centrée sur 10 µm fait son apparition, suivie d'une 3^{ème} population composée de gros agglomérats de diamètres supérieurs au mm (très gros globules gras totalement instables).

Il apparaît que l'ajout d'émulsifiant a un effet très négatif sur la finesse et l'homogénéité de l'émulsion.

## Revendications

1. Procédé de préparation d'une émulsion stable huile dans eau, dans lequel on combine une phase lipidique ou lipophile avec une phase aqueuse, **caractérisé en ce qu'**on soumet le mélange à une énergie vibratoire, dans un récipient fermé, par application d'un transducteur opérant dans une plage de fréquences comprises entre 900 kHz et 3 MHz.

2. Procédé selon la revendication 1, **caractérisé en ce que** le traitement vibratoire est appliqué dans des conditions essentiellement sans cavitation.

3. Procédé selon la revendication 1 ou 2, **caractérisé en ce que** le transducteur opère à une fréquence inférieure à 1 500 kHz.

4. Procédé selon la revendication 1, 2 ou 3, **caractérisé en ce que** le transducteur opère à une fréquence supérieure à 1 MHz.

5. Procédé selon l'une quelconque des revendications précédentes, **caractérisé en ce que** le mélange, respectivement l'émulsion, est exempt(e) d'émulsifiant ajouté.

6. Procédé selon l'une quelconque des revendications précédentes, **caractérisé en ce que** le transducteur est de type piézo-électrique.

7. Procédé selon l'une quelconque des revendications précédentes, **caractérisé en ce que** les vibrations sont appliquées au mélange dans la partie inférieure du récipient fermé contenant le mélange.

8. Procédé selon l'une quelconque des revendications précédentes, **caractérisé en ce que** la part de phase lipidique n'excède pas 40 %, de préférence 30 %, ou mieux 20 % du poids total.

9. Procédé selon l'une quelconque des revendications précédentes, **caractérisé en ce qu'**on maintient le mélange dans une plage de température prédéterminée, de préférence dans une plage de température entre 20 et 30°C.

10. Procédé selon l'une quelconque des revendications précédentes, **caractérisé en ce que** le traitement est continué jusqu'à dispersion complète de la phase lipidique et/ou obtention de la concentration finale désirée en phase lipidique et/ou de la granulométrie finale désirée.

11. Procédé selon l'une quelconque des revendications précédentes, **caractérisé en ce que** le traitement est continué jusqu'à obtention d'une granulométrie micrométrique, de préférence jusqu'à obtention d'une phase discontinue présentant des particules d'un diamètre moyen inférieur à 50 µm, de préférence inférieur à 20 µm.

12. Procédé selon l'une quelconque des revendications précédentes, **caractérisé en ce que** l'émulsion obtenue est stable pendant plusieurs semaines à plusieurs mois à température ambiante.

13. Procédé selon l'une quelconque des revendications précédentes, **caractérisé en ce que** la phase lipidique ou lipophile est choisie parmi le groupe comprenant : les huiles végétales, les huiles animales, les beurres, les huiles d'avocat ou d'argan, huiles essentielles.

14. Utilisation du procédé selon l'un quelconque des revendications 1 à 10 pour la préparation de compositions dermatologiques ou pharmaceutiques ou de produits alimentaires.

## Patentansprüche

1. Verfahren zur Herstellung einer stabilen Öl-in-Wasser-Emulsion, wobei eine Fett- oder Lipophilphase mit einer Wasserphase kombiniert wird, **dadurch gekennzeichnet, dass** das Gemisch in einem geschlossenen Behälter durch Anwendung eines Wandlers, der in einem Frequenzbereich zwischen 900 kHz und 3 MHz arbeitet, einer Vibrationsenergie unterworfen wird.

2. Verfahren nach Anspruch 1, **dadurch gekennzeichnet, dass** die Vibrationsbehandlung in Bedingungen im Wesentlichen ohne Kavitation angewendet wird.

3. Verfahren nach Anspruch 1 oder 2, **dadurch gekennzeichnet, dass** der Wandler mit einer Frequenz kleiner als 1500 kHz arbeitet.

4. Verfahren nach Anspruch 1, 2 oder 3, **dadurch gekennzeichnet, dass** der Wandler mit einer Frequenz höher als 1 MHz arbeitet.

5. Verfahren nach einem der vorhergehenden Ansprüche, **dadurch gekennzeichnet, dass** das Gemisch bzw. die Emulsion frei von zugegebenem Emulgator ist.

6. Verfahren nach einem der vorhergehenden Ansprüche, **dadurch gekennzeichnet, dass** der Wandler vom piezoelektrischen Typ ist.

7. Verfahren nach einem der vorhergehenden Ansprüche, **dadurch gekennzeichnet, dass** die Vibrationen auf das Gemisch im unteren Teil des geschlossenen Behälters, der das Gemisch enthält, angewendet werden.

8. Verfahren nach einem der vorhergehenden Ansprüche, **dadurch gekennzeichnet, dass** der Teil der Fettphase 40 %, vorzugsweise 30 % oder am besten 20 % des Gesamtgewichts nicht übersteigt.

9. Verfahren nach einem der vorhergehenden Ansprüche, **dadurch gekennzeichnet, dass** das Gemisch in einem vorher festgelegten Temperaturbereich, vorzugsweise in einem Temperaturbereich zwischen 20 und 30 °C gehalten wird.

10. Verfahren nach einem der vorhergehenden Ansprüche, **dadurch gekennzeichnet, dass** die Behandlung bis zur vollständigen Dispersion der Fettphase und/oder Erzielung der gewünschten Endkonzentration der Fettphase und/oder der gewünschten Endgranulometrie fortgesetzt wird.

11. Verfahren nach einem der vorhergehenden Ansprüche, **dadurch gekennzeichnet, dass** die Behandlung bis zur Erzielung einer mikrometrischen Granulometrie, vorzugsweise bis zur Erzielung einer diskontinuierlichen Phase, die Teilchen mit einem durchschnittlichen Durchmesser kleiner als 50 µm, vorzugsweise kleiner als 20 µm aufweist, fortgesetzt wird.

12. Verfahren nach einem der vorhergehenden Ansprüche, **dadurch gekennzeichnet, dass** die erhaltene Emulsion über mehrere Wochen bis mehrere Monate bei Umgebungstemperatur stabil ist.

13. Verfahren nach einem der vorhergehenden Ansprüche, **dadurch gekennzeichnet, dass** die Fett- oder Lipophilphase aus der Gruppe umfassend: Pflanzenöle, Tierische Öle, Buttern, Avocadoöl, Arganöl, ätherische Öle, ausgewählt ist.

14. Verwendung des Verfahrens nach einem der Ansprüche 1 bis 10 zur Herstellung von dermatologischen oder pharmazeutischen Zusammensetzungen oder Nahrungsmitteln.

## Claims

1. A method of preparing a stable oil-in-water emulsion, in which a lipid or lipophil phase is combined with an aqueous phase, **characterised in that** the mixture is exposed to vibratory energy, in a closed receptacle, by application of a transducer operating in a frequency range of between 900 kHz and 3 MHz.

2. The method according to claim 1, **characterised in that** the vibratory treatment is applied under substantially cavitation-free conditions.

3. The method according to claim 1 or claim 2, **characterised in that** the transducer operates at a frequency of less than 1 500 kHz.

4. The method according to claim 1, 2 or 3, **characterised in that** the transducer operates at a frequency greater than 1 MHz.

5. The method according to any one of the preceding claims, **characterised in that** the mixture, or emulsion, contains no added emulsifier.

6. The method according to any one of the preceding claims, **characterised in that** the transducer is of the piezoelectric type.

7. The method according to any one of the preceding claims, **characterised in that** the vibrations are applied to the mixture in the lower part of the closed receptacle containing the mixture.

8. The method according to any one of the preceding claims, **characterised in that** the lipid phase constitutes no more than 40 %, preferably 30 %, or better 20 % of the total weight.

9. The method according to any one of the preceding claims, **characterised in that** the mixture is kept within a predetermined temperature range, preferably in a temperature range of between 20 and 30°C.

10. The method according to any one of the preceding claims, **characterised in that** the treatment is continued until the lipid phase is completely dispersed and/or the final desired lipid phase concentration and/or the final desired particle size is/are obtained.

11. The method according to any one of the preceding claims, **characterised in that** the treatment is continued until a micrometric particle size is obtained, preferably until a discontinuous phase is obtained which has particles of an average diameter of less than 50 µm, preferably less than 20 µm.

12. The method according to any one of the preceding claims, **characterised in that** the emulsion obtained is stable for several weeks to several months at ambient temperature.

13. The method according to any one of the preceding claims, **characterised in that** the lipid or lipophil phase is selected from the group comprising: vegetable oils, animal oils, butters, avocado oil, argan oil, essential oils.

14. Use of the method according to any one of claims 1 to 10 for preparing dermatological or pharmaceutical compositions or food products.
